# EUROPÄISCHE PATENTSCHRIFT

(11) **EP 1 223 158 B1**
(45) Veröffentlichungstag und Bekanntmachung des Hinweises auf die Patenterteilung: **11.08.2004**
(21) Anmeldenummer: 01131039.8
(22) Anmeldetag: 28.12.2001
(51) Int. Cl.: C07C 51/08, C07C 63/331, C07C 255/50

(54) **Verfahren zur Herstellung trifluormethyl-substituierter Biphenylcarbonsäuren und neue trichlormethyl- und trifluormethyl-substituierte Biphenylcarbonitrile**
Process for preparing trifluoromethyl substituted biphenyl carboxylic acids and novel trichloromethyl and trifluoromethyl substituted biphenyl nitriles
Procédé de préparation d'acides biphényl carboxyliques substitués par un groupe trifluoromethyl et nouveaux nitriles de biphényl substitués par un groupe trifluoro- ou trichloro- methyl

(30) Priorität: 12.01.2001 DE 10101150
(43) Veröffentlichungstag der Anmeldung: 17.07.2002
(73) Patentinhaber: Bayer Chemicals AG, 51368 Leverkusen (DE)
(72) Erfinder: Müller, Peter, Dr., 51519 Odenthal (DE); Marhold, Albrecht, Dr., 51373 Leverkusen (DE); Peilstöcker, Karen, Dr., 51069 Köln (DE)

(56) Entgegenhaltungen:
- EP-A- 0 059 983
- MATSUHISA A ET AL: "NONPEPTIDE ARGININE VASOPRESSIN ANTAGONISTS FOR BOTH V1A AND V2 RECEPTORS: SYNTHESIS AND PHARMACOLOGICAL PROPERTIES OF 2-PHENYL-4'-(2,3,4,5-TETRAHYDRO-1H-1-BENZA ZEPIN-1-YL)CARBONYLBENZANI LIDE DERIVATIVES" CHEMICAL AND PHARMACEUTICAL BULLETIN, PHARMACEUTICAL SOCIETY OF JAPAN. TOKYO, JP, Bd. 45, Nr. 11, 1997, Seiten 1870-1874, XP000887167 ISSN: 0009-2363
- OHKAWA ET AL.: "Synthesis and characterization of orally active non peptide vasopressin V2 receptor antagonists " CHEM. PHARM. BULL., Bd. 47, Nr. 4, 1999, Seiten 501-510, XP001056586
- CARINI D J ET AL: "NONPEPTIDE ANGIOTENSIN II RECEPTOR ANTAGONISTS: THE DISCOVERY OF A SERIES OF N-(BIPHENYLYLMETHYL)IMIDAZOLES AS POTENT, ORALLY ACTIVE ANTIHYPERTENSIVES" JOURNAL OF MEDICINAL CHEMISTRY, AMERICAN CHEMICAL SOCIETY. WASHINGTON, US, Bd. 34, Nr. 8, 1. August 1991 (1991-08-01), Seiten 2525-2547, XP000674205 ISSN: 0022-2623

## Beschreibung

Die vorliegende Erfindung betrifft ein Verfahren zur Herstellung von trifluormethyl-substituierten Biphenylcarbonsäuren aus den entsprechenden methyl-substituierten Biphenylcarbonitrilen und dabei als Zwischenprodukte auftretende neue trichlormethyl-substituierte Biphenylcarbonitrile und neue trifluormethyl-substituierte Biphenylcarbonitrile.

Trifluormethyl-substituierte Biphenylcarbonsäuren sind Zwischenprodukte für die Herstellung pharmazeutischer Wirkstoffe. 4'-(Trifluormethyl)-2-biphenylcarbonsäure selbst ist ein als Xenalipin bekannter pharmazeutischer Wirkstoff (siehe auch: Matsuhisa A. et al, Bull. Pharm. Soc. Jap., Bd. 45, Nr. 11, 1997, Seiten 1870-1874; Ohkawa et al, Chern. Pharm. Bull., Bd. 47, Nr. 4, 1999, Seiten 501-510; Carini D. J. et al, Journ. Med. Chem., Am. Chem. Soc., Bd. 34, Nr. 8, Seiten 2525-2547).

Es ist bekannt, dass man trifluormethyl-substituierte Biphenylcarbonsäuren herstellen kann durch Aufbau des trifluormethyl-substituierten Biphenylgerüsts mittels einer Arylkupplung geeigneter Benzotrifluorid-Derivate, wobei Palladium-, Nickel-, Zink-, oder Grignard-Reagenzien zu verwenden sind und die Carboxylfunktion eines Vorläufers (z.B. Aldehyd oder Ester) mit einer geeigneten Schutzgruppe blockiert in dem entsprechenden Kupplungspartner vorliegt und anschließend an die Kupplung die Carbonsäurefunktion durch Transformation des Carboxylvorläufers oder Abspaltung der Schutzgruppe hergestellt wird (siehe beispielsweise Organ. Prep. Proced. Int., 27(3), 367 (1995), WO 00/32582 und EP-A 59983).

Für den technischen Maßstab sind diese Verfahren wenig geeignet, weil in allen Fällen metallorganische Verbindungen hergestellt und gehandhabt werden müssen, was nur mit großem technologischem Aufwand möglich ist. Außerdem kommen je nach Kupplungsmethode teure und/oder nur aufwendig herzustellende Benzotrifluorid-Derivate oder substituierte Benzoesäure-Derivate zum Einsatz. Je nach Wahl des Carboxyl-Vorläufers müssen zusätzlich Schutzgruppen eingeführt und wieder abgespalten werden.

Es besteht daher noch ein Bedürfnis nach einem ohne besonderen Aufwand, ausgehend von einfacher zugänglichen Ausgangsprodukten, sicher im technischen Maßstab durchführbaren Verfahren zur Herstellung von trifluormethyl-substituierten Biphenylcarbonsäuren.

Es wurde nun ein Verfahren zur Herstellung von trifluormethyl-substituierten Biphenylcarbonsäuren der Formel (I) gefunden 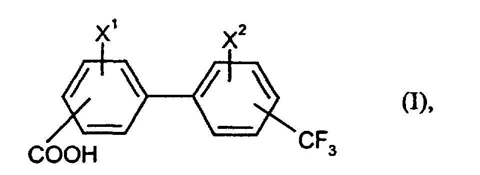 in der
X¹ und X² gleich oder verschieden sind und jeweils für Wasserstoff, Chlor oder Fluor stehen,
das dadurch gekennzeichnet ist, dass man methyl-substituierte Biphenylcarbonitrile der Formel (IV) 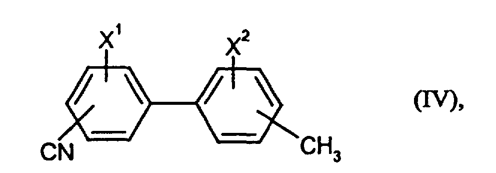 in der
X¹ und X² die bei Formel (I) angegebene Bedeutung haben,
in die entsprechenden trichlormethyl-substituierten Biphenylcarbonitrile der Formel (III) umwandelt 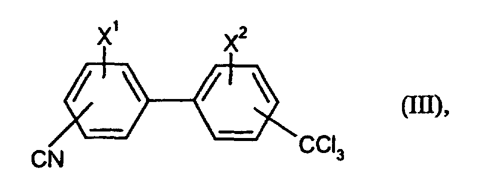 in der
X¹ und X² die bei Formel (I) angegebene Bedeutung haben,
deren Trichlormethylgruppe in eine Trifluormethylgruppe überführt und so trifluormethyl-substituierte Biphenylcarbonitrile der Formel (II) erhält 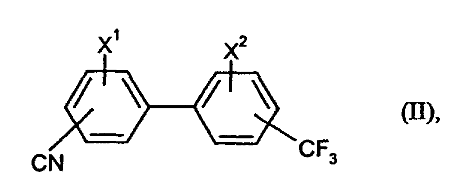 in der
X¹ und X² die bei Formel (I) angegebene Bedeutung haben,
und abschließend daraus durch Hydrolyse die entsprechenden Carbonsäuren erhält.

In den Formeln (I) bis (IV) stehen X¹ und X² bevorzugt für Wasserstoff. Die Trifluormethylgruppe in den Formeln (I) und (II), die Trichlormethylgruppe in Formel (III) und die Methylgruppe in Formel (IV) befinden sich bevorzugt para-ständig zur Biphenylverknüpfung. Die Carboxylgruppe in Formel (I) und die Nitrilgruppe in den Formeln (II), (III) und (IV) sind bevorzugt ortho-ständig zur Biphenylverknüpfung. Besonders bevorzugt wird erfindungsgemäß 4'-(Trifluormethyl)-2-biphenylcarbonsäure aus 4'-Methyl-2-biphenylcarbonitril hergestellt.

Die erste Stufe des erfindungsgemäßen Verfahrens, die Herstellung der trichlormethyl-substituierten Biphenylcarbonitrile der Formel (III) kann beispielsweise in Form einer radikalischen Seitenkettenchlorierung von entsprechenden methyl-substiuierten Biphenylcarbonitrilen der Formel (IV) durchgeführt werden, bei der man erhöhte Temperatur, Bestrahlung mit einer Lichtquelle und/oder den Zusatz eines Radikalstarters anwendet. Als Lichtquellen eignen sich insbesondere Halogenlampen und Mitteldruck- und Hochdruck-Quecksilberlampen. Als Radikalstarter kommen beispielsweise Benzoylperoxid, Di-tert.-butylperoxid, 2,2'-Aza-bis-(isobuttersäurenitril), 2-Phenylazo-2,4-dimethyl-4-methoxy-valeronitril, tert.-Butylperoxy-2-ethylhexanoat und andere in Frage. Bevorzugt setzt man eine Lichtquelle bei erhöhter Temperatur ein.

Die Reaktionstemperatur kann z.B. zwischen 80°C und 250°C liegen. Vorzugsweise liegt sie bei 100°C bis 200°C, insbesondere bei 110°C bis 160°C.

Als Chlorierungsmittel für die erste Stufe des erfindungsgemäßen Verfahrens verwendet man vorzugsweise elementares Chlor. Es können gegebenenfalls auch andere Chlorierungsmittel eingesetzt werden, die sich für radikalische Seitenkettenchlorierungen eignen.

Pro Mol methyl-substituiertes Biphenylcarbonitril der Formel (IV) kann man beispielsweise 2 bis 10 Äquivalente, vorzugsweise 4 bis 7 Äquivalente Chlorierungsmittel einsetzen.

Die radikalische Seitenkettenchlorierung wird vorzugsweise in Gegenwart von Lösungsmitteln durchgeführt. Lösungsmittel sind dann zwingend erforderlich, wenn es sich bei den eingesetzten methyl-substituierten Biphenylcarbonitrilen der Formel (IV) um bei Reaktionsbedingungen feste Stoffe handelt. Als Lösungsmittel kommen beispielsweise halogenierte Kohlenwasserstoffe wie Chlorbenzol, Dichlorbenzole und Trichlorbenzole, halogenierte Benzotrifluoride wie 4-Chlor-benzotrifluorid, halogenierte Bis-(trifluormethyl)-benzole und Phosphoroxychlorid in Frage. Pro g methyl-substituiertes Biphenylcarbonitril der Formel (IV) kann man beispielsweise 0,5 g bis 2,5 g Lösungsmittel einsetzen. Bevorzugte Lösungsmittel sind 2-Chlor- und 4-Chlor-benzotrifluorid.

Die radikalische Seitenkettenchlorierung kann man gegebenenfalls gaschromatographisch verfolgen und vorzugsweise so lange durchführen, bis das eingesetzte methyl-substituierte Biphenylcarbonitril der Formel (IV) möglichst weitgehend zum gewünschten Produkt umgesetzt ist.

Das nach Durchführung der ersten Stufe vorliegende Reaktionsgemisch kann man beispielsweise aufarbeiten, indem man zunächst noch vorhandenes Chlor entfernt, z.B. durch Einleiten eines Inertgases oder Anlegen von Vakuum. Das dann vorliegende Rohprodukt kann direkt in die nächste Reaktionsstufe eingesetzt werden. Wenn gewünscht kann man das erhaltene trichlormethyl-substituierte Biphenylcarbonitril der Formel (III) auch weiter reinigen, beispielsweise durch eine Kristallisation oder eine Vakuumdestillation. Im Rahmen des erfindungsgemäßen Verfahrens wird bevorzugt das nach dem Abtrennen des überflüssigen Chlors vorhandene Rohmaterial weiter verarbeitet.

Die zweite Stufe des erfindungsgemäßen Verfahrens, die Umwandlung eines trichlormethyl-substituierten Biphenylcarbonitrils der Formel (III) in die entsprechende Trifluormethyl-Verbindung kann beispielsweise durch Umsetzung mit einem Fluorierungsmittel wie wasserfreie Flusssäure gegebenenfalls in Gegenwart eines Fluorierungskatalysators wie Antimonpentafluorid, Antimonpentachlorid, Bortrifluorid oder Titantetrachlorid durchgeführt werden. Vorzugweise setzt man hierzu wasserfreie Flusssäure ein.

Pro Mol trichlormethyl-substituiertes Biphenylcarbonitril der Formel (III) kann man beispielsweise 200 bis 500 ml wasserfreie Flusssäure oder eine entsprechende Menge eines anderen Fluorierungsmittels einsetzen. Die Menge an Fluorierungskatalysator kann beispielsweise 0 bis 0,2 Mol pro Mol trichlormethyl-substituiertes Biphenylcarbonitril betragen.

Die Fluorierung kann man beispielsweise so durchführen, dass man bei einer Temperatur unterhalb des Siedepunktes (bei Normaldruck) von Fluorwasserstoff beginnt, beispielsweise bei -20°C bis +15°C und die Fluorierung bei höheren Temperaturen beispielsweise bei 50°C bis 150°C zu Ende führt. Durch den Dampfdruck des Fluorwasserstoffs bei höheren Temperaturen können dabei Drücke bis zu beispielsweise 100 bar auftreten, was die Verwendung entsprechend druckfester Reaktionsgefäße erforderlich macht. Den freiwerdenden Chlorwasserstoff kann man beispielsweise bei Temperaturen über +20°C über ein Druckhalteventil entspannen.

Zur Aufarbeitung des Reaktionsgemisches kann man nach Beendigung der Reaktion, Abkühlung und Entspannung des Chlorwasserstoffs beispielsweise zunächst überschüssige Flusssäure entfernen, z.B. durch Destillation. Die weitere Reinigung des hergestellten trifluormethyl-substituierten Biphenylcarbonitrils der Formel (II) kann beispielsweise durch Destillation, Kristallisation oder Gelchromatographie erfolgen.

Bei der abschließenden dritten Stufe des erfmdungsgemäßen Verfahrens wird die Nitrilgruppe vorzugsweise alkalisch hydrolysiert und die entsprechende Carboxylverbindung erhalten. Die Hydrolyse kann man beispielsweise mit einer wässrigen alkalischen Lösung durchführen, vorzugsweise mit wässriger Kali- oder Natronlauge. Pro Mol trifluormethyl-substituiertes Biphenylcarbonitril der Formel (II) kann man beispielsweise 2,5 bis 6 Mol Alkalihydroxid in Form einer wässrigen Lösung von Konzentrationen im Bereich von beispielsweise 5 bis 50 Gew.-% einsetzen.

Die Hydrolyse kann man beispielsweise so durchführen, dass man das trifluormethyl-substituierte Biphenylcarbonitril der Formel (II) zusammen mit einer wässrigen Alkalihydroxid-Lösung in einem druckfesten Reaktionsgefäß auf Temperaturen von beispielsweise 130°C bis 200°C für beispielsweise 5 bis 40 Stunden erhitzt. Dabei können Drucke bis zu beispielsweise 100 bar auftreten, was die Verwendung entsprechender druckfester Reaktionsgefäße erforderlich macht.

Die Aufarbeitung des nach der alkalischen Hydrolyse vorliegenden Reaktionsgemisches kann man z.B. so durchführen, dass man zunächst gegebenenfalls vorhandene feste Bestandteile abtrennt, z.B. durch Filtration, vorzugsweise nach Zusatz eines Filterhilfsmittels wie Cellulose, Celite® oder ein Zeolith.

Zur weiteren Reinigung kann man das wässrige Filtrat mit einem nicht mit Wasser mischbaren organischen Lösungsmittel extrahieren. Dafür kommen beispielsweise aliphatische, alicyclische und aromatische Kohlenwasserstoffe in Betracht wie Petrolether, Hexan, Heptan, Cyclohexan, Benzol, Toluol und Xylole sowie mit Wasser nicht mischbare Ether wie Diethylether, Methyl-tert.-butylether, 1,2-Dimethoxyethan und 1,2-Diethoxyethan.

Aus der wässrigen Phase können anschließend beispielsweise durch Zusatz einer Säure wie wässrige Salzsäure oder Schwefelsäure, die trifluormethyl-substituierten Biphenylcarbonsäuren der Formel (I) erhalten werden. Um besonders reines Produkt der Formel (I) zu erhalten, ist es vorteilhaft, den pH-Wert bei Zugabe der Säure zur alkalischen Reaktionslösung auf einen Wert von beispielsweise 7 bis 1, bevorzugt auf einen Wert von 7 bis 6, einzustellen. Das ausgefallene Produkt kann dann z.B. durch Filtration isoliert werden.

Mit dem erfindungsgemäßen Verfahren lassen sich trifluormethyl-substituierte Biphenylcarbonsäuren der Formel (I) aus den gut, teilweise auch kommerziell zugänglichen methyl-substituierten Biphenylcarbonitrilen der Formel (IV) in einem technisch gut und einfach durchzuführenden Verfahren in guten Ausbeuten herstellen. Über alle Reaktionsstufen betrachtet, liegen die Ausbeuten deutlich höher als 65 % der Theorie.

Die Verbindungen der Formel (I), (II) und (III) sind teilweise neu. Die vorliegende Erfindung betrifft deshalb auch trifluormethyl-substituierte Biphenylcarbonitrile der Formel (II) und trichlormethyl-substituierte Biphenylcarbonitrile der Formel (III), bei denen X¹ und X² gleich oder verschieden sind und jeweils für Chlor oder Fluor stehen. Die bevorzugte Stellung der Substituenten am Biphenylgerüst ist wie oben angegeben. Die Herstellung der neuen Verbindungen ist ebenfalls oben beschrieben.

Bei den neuen Verbindungen handelt es sich um neue Zwischenprodukte zur Herstellung von trifluormethyl-substituierten Biphenylcarbonsäuren der Formel (I) nach dem erfindungsgemäßen Verfahren. Mit ihnen kann die Palette von Zwischenprodukten auf der Basis von trifluormethyl-substituierten Biphenylcarbonsäuren erweitert werden und damit vergrößern sich die Möglichkeiten zur Herstellung von Produkten, die auf ihre Geeignetheit als Pharmawirkstoffe getestet werden können.

### Beispiele

### Beispiel 1

### 2-Cyano-4'-trichlormethyl-biphenyl

In einem 10-l Glaskolben wurden 5 040 g 2-Cyano-4'-methyl-biphenyl in 4 000 g 4-Chlor-benzotrifluorid vorgelegt und unter Rühren auf 130°C erhitzt. Unter Bestrahlung des Reaktionsgefäßes mit einer UV-Lampe wurden in 33 Stunden 7 430 g Chlorgas eingeleitet. Nun wurde noch vorhandenes Chlor mit Stickstoff ausgeblasen, wobei 11 560 g einer Mischung hinterblieben, die zu 30,8 % (GC, Flächenprozent) 4-Chlor-benzotrifluorid und 67 % (GC, quantitativ) 2-Cyano-4'-trichlormethyl-biphenyl enthielt. Nach dem Einengen des Reaktionsgemisches im Vakuum fiel nach 2,5 Tagen 2-Cyano-4'-trichlormethyl-biphenyl aus (Schmelzpunkt 67°C bis 68°C). Das ¹H-NMR-Spektrum enthielt folgende charakteristischen Absorptionen (δ/ppm, CDCl₃): 7,52 (ddd, 1H, J = 7,6 Hz, J = 7,7 Hz, J = 1,3 Hz, H-4); 7,54 (dd, 1H, J = 7,6 Hz, J = 1,3 Hz, H-6); 7,64 (d, 2H, J = 8,8 Hz, H-2', H-6'); 7,69 (ddd, 1H, J = 7,7 Hz, J = 1,4 Hz, H-5); 7,81 (dd, 1H, J = 7,7 Hz, J = 1,4 Hz, H-3); 8,06 (d, 2H, J = 8,8 Hz, H-3', H-5').

### Beispiel 2

### 2-Cyano-4'-trifluormethyl-biphenyl

In einem 5-l-Edelstahl-Rührautoklaven mit aufsteigendem Kühler (betrieben mit einem Kühlmittel von -10°C) und Druckregler wurden 1 600 ml wasserfreie Flusssäure vorgelegt. Dann tropfte man innerhalb von 50 min bei -2 bis 0°C 2 100 g des gemäß Beispiel 1 erhaltenen Gemisches zu. Es setzte eine schwache Entwicklung von Chlorwasserstoff ein. Die Temperatur wurde dann auf 19°C ansteigen gelassen, 3 bar Stickstoff aufgedrückt, auf 60°C erwärmt und 5 Stunden bei 60°C nachgerührt. Danach wurde der Autoklav abgekühlt, entspannt und der überschüssige Fluorwasserstoff im Vakuum abdestilliert. Der Rückstand wurde auf 3 000 g Eiswasser geschüttet und durch Zugabe von 2 700 ml n-Hexan ausgefällt.

Der so erhaltene Niederschlag wurde abfiltriert, zweimal mit n-Hexan gewaschen und getrocknet. So wurden 735 g (63 % der Theorie) eines hellbeigen Feststoffes mit einer Reinheit von 98,9 % (GC, Flächenprozent), Schmelzpunkt 101°C bis 102°C erhalten. Eine weitere Menge an Produkt konnte erhalten werden, indem aus dem Filtrat die organische Phase abgetrennt und auf ein Drittel ihres Volumens im Vakuum eingeengt wurde. Das Versetzen mit 500 ml n-Hexan ergab eine weitere Produktausfällung, die abfiltriert, mit n-Hexan gewaschen und getrocknet wurde. Es wurden so weitere 161 g (14 % der Theorie) eines hellbeigen Feststoffes der Reinheit 99,2 % (GC, Flächenprozent), Schmelzpunkt 101°C bis 102°C erhalten.

Eine weitere entsprechende Behandlung des nunmehr vorliegenden Filtrats ergibt weiteres Produkt, so dass dieses insgesamt in einer Ausbeute von 86 % der Theorie erhalten wurde.

### Beispiel 3

### 4'-(Trifluormethyl)-2-biphenylearbonsäure

113 g 2-Cyano-4'-trifluormethyl-biphenyl wurden mit 587 g 14 gew.-%iger wässriger Kaliumhydroxid-Lösung versetzt und 24 Stunden bei 160°C in einem Autoklaven unter Eigendruck gerührt. Danach wurde der Autoklav abgekühlt und das wässrige Reaktionsgemisch dreimal mit Toluol extrahiert. Die wässrige Phase wurde anschließend mit wässriger Salzsäure auf einen pH-Wert im Bereich 7 bis 6 eingestellt. Der ausfallende Niederschlag wurde abfiltriert und getrocknet. Es wurden 117 g (95 % der Theorie) eines hellbraunen Feststoffs in einer Reinheit von 97,2 % (HPLC, Flächenprozent) mit einem Schmelzpunkt von 169°C bis 170°C erhalten.

### Beispiel 4

### 2-Cyano-3'-chlor-4'-trichlormethyl-biphenyl

In einem 1-Liter-Glaskolben werden 180 g 2-Cyano-3'-chlor-4'-methyl-biphenyl in 180 g 4-Chlorbenzotrifluorid vorgelegt und unter Rühren auf 120° bis 140°C erhitzt. Unter Bestrahlung des Reaktionsgefäßes mit einer UV-Lampe werden innerhalb von 31 Stunden 2 114 g Chlorgas eingeleitet. Nach Ausblasen des Chlorüberschusses mit Stickstoff hinterbleiben 421 g einer Mischung aus 40,4 % (GC-Flächenprozent) 4-Chlorbenzotrifluorid und 43,6 % (GC) 2-Cyano-3'-chlor-4'-trichlornlethyl-biphenyl. Das Gemisch wird als Rohprodukt in die Fluorierung eingesetzt.

Das ¹H-NMR-Spektrum enthielt folgende charakteristische Absorptionen (CDCl₃, δ/ppm): 8.32 (d, 1H, J = 8.36 Hz, H-5'); 7,84 (dd, 1H, J = 1,3 Hz, J = 8,07 Hz, H-3); 7,75 (d, 1H, J = 1,98 Hz, H-2'); 7,73 (dt, 1H, J = 1,35 Hz, J = 7,62 Hz, H-5); 7,61 (d, 1H, J = 1,98 Hz, H-6'); 7,56 (dt, 1H, J = 1,2 Hz, J = 6,48 Hz, H-4); 7,55 (d, 1H, J = 7,8 Hz, H-6); Mittels GC-MS erhält man folgendes Spektrum (EI, 70eV, I/%): 331 (5,0, M⁺); 296 (100, M-Cl)⁺); 260 (11, M-2 Cl)⁺); 224 (20, (M-3Cl)⁺); 188 (13, (M-4Cl)⁺).

### Beispiel 5

### 2-Cyano-3'-chlor-4'-trifluormethyl-biphenyl

381 g einer 43,6 %igen Lösung (GC) von 2-Cyano-3'-chlor-4'-trichlormethyl-biphenyl werden in einem 2-l-Edelstahl-Rührautoklaven mit aufsteigendem Kühler (betrieben mit einem Kühlmittel von -10°C) und Druckregler vorgelegt. Bei 0°C lässt man 300 ml wasserfreie Flusssäure zulaufen und rührt anschließend 22 Stunden bei Raumtemperatur. Nach beendeter Chlorwasserstoffentwicklung werden 5 bar Stickstoff aufgedrückt, auf 60°C erwärmt und 4 Stunden bei 60°C reagieren lassen. Der Autoklav wird abgekühlt, entspannt und der überschüssige Fluorwasserstoff wird im Vakuum abdestilliert. Der Rückstand wird zuerst vorsichtig mit 500 ml Eiswasser und danach mit 2 l Dichlormethan versetzt. Die organische Phase wird abgetrennt, getrocknet und im Vakuum eingeengt. Das Rohprodukt wird durch Versetzen des Rückstandes mit 1 Liter n-Hexan unter starkem Rühren erhalten und wird anschließend aus 1 Liter n-Hexan umkristallisiert. Nach dem Trocknen erhält man 120 g (71 % der Theorie) eines hellbraunen Feststoffs vom Schmelzpunkt 138 bis 140°C.

Das ¹H-NMR-Spektrum enthielt folgende charakteristische Absorptionen (CDCl₃, δ/ppm): 7,86 (d, 1H, J = 8,15 Hz, H-3); 7,85 (ddd, 1H, J = 0,5 Hz, J = 1,58 Hz, J = 7,79 Hz, H-5'); 7,74 (dt, 1H, J = 1,38 Hz, J = 7,72 Hz, H-5); 7,71 (m, 1H, H-2'); 7,61 (m, 1H, H-6'); 7,58 (dt, 1H, J = 1,25 Hz, J = 7,68 Hz, H-4); 7,55 (ddd, 1H, J = 0,54 Hz, 1,24 Hz, 7,81 Hz, H-6). Mittels GC-MS erhält man folgendes Spektrum (EI, 70eV, I/%): 281 (100, M⁺); 246 (35, (M-Cl)⁺); 226 (29, (M-Cl-HF)⁺).

### Beispiel 6

### 4'-(Trifluormethyl)-3'-chlor-2-biphenylcarbonsäure

60 g 2-Cyano-3'-chlor-4'-trifluormethyl-biphenyl werden mit einer Lösung von 38,7 g Kaliumhydroxid in 325 ml Wasser versetzt und 24 Stunden bei 160°C in einem Autoklaven unter Eigendruck gerührt. Danach wird der Autoklav abgekühlt, das wässrige Reaktionsgemisch über Celite filtriert und anschließend zweimal mit je 200 ml Toluol extrahiert. Die wässrige Phase wird mit 10 %iger Salzsäure auf einen pH-Wert von 6 bis 7 gebracht. Der ausgefallene Feststoff wird abfiltriert, mit Wasser gewaschen und getrocknet. Es wurden 30 g (47 % der Theorie) eines hellbeigen Feststoffs vom Schmelzpunkt 181 bis 184°C in einer Reinheit von 92,7 % (HPLC, Flächenprozent) erhalten.

Das ¹H-NMR-Spektrum enthielt folgende charakteristische Absorptionen (MeOHd4, δ/ppm): 7,97 (dd, 1H, J = 7,78 Hz, J = 1,41 Hz, H-3); 7,79 (d, 1H, J = 8,07 Hz, H-5'); 7,64 (dt, 1H, J = 1,41 Hz, J = 7,56 Hz, H-5); 7,56 (m, 1H, H-2'); 7,54 (dt, 1H, J = 1,33 Hz, J = 7,57 Hz, H-4); 7,43 (m, 1H, H-6'); 7,39 (dd, 1H, J = 1,29 Hz, J = 7,65 Hz, H-6).

## Patentansprüche

1. Verfahren zur Herstellung von trifluormethyl-substituierten Biphenylcarbonsäuren der Formel (I) 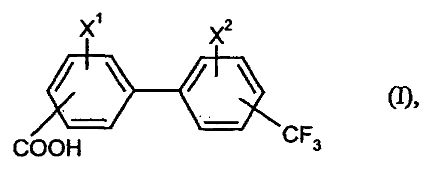 in der
X¹ und X² gleich oder verschieden sind und jeweils für Wasserstoff, Chlor oder Fluor stehen,
**dadurch gekennzeichnet, dass** man methyl-substituierte Biphenylcarbonitrile der Formel (IV) 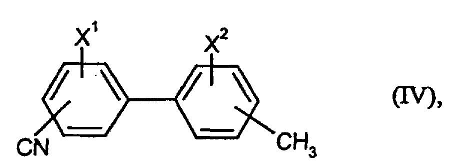 in der
X¹ und X² die bei Formel (I) angegebene Bedeutung haben,
in die entsprechenden trichlormethyl-substituierten Biphenylcarbonitrile der Formel (III) umwandelt 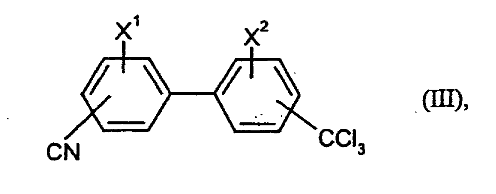 in der
X¹ und X² die bei Formel (I) angegebene Bedeutung haben,
deren Trichlormethylgruppe in eine Trifluormethylgruppe überführt und so trifluormethyl-substituierte Biphenylcarbonitrile der Formel (II) erhält 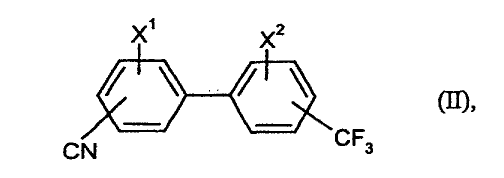 in der
X¹ und X² die bei Formel (I) angegebene Bedeutung haben,
und abschließend daraus durch Hydrolyse die entsprechenden Carbonsäuren erhält.

2. Verfahren nach Anspruch 1, **dadurch gekennzeichnet, dass** in den Formeln (I) bis (IV) X¹ und X² für Wasserstoff stehen.

3. Verfahren nach Ansprüchen 1 und 2, **dadurch gekennzeichnet, dass** die Trifluormethylgruppe in den Formeln (I) und (II), die Trichlormethylgruppe in Formel (III) und die Methylgruppe in Formel (IV) sich para-ständig zur Biphenylverknüpfung und die Carboxylgruppe in Formel (I) und die Nitrilgruppe in den Formeln (II), (III) und (IV) sich ortho-ständig zur Biphenylverknüpfung befinden.

4. Verfahren nach Ansprüchen 1 bis 3, **dadurch gekennzeichnet, dass** man die Herstellung der trichlormethyl-substituierten Biphenylcarbonitrile der Formel (III) in Form einer radikalischen Seitenkettenchlorierung bei Temperaturen von 80 bis 250°C, unter Bestrahlung mit einer Lichtquelle und/oder dem Zusatz eines Radikalstarters und unter Verwendung von Chlor als Chlorierungsmittel durchführt.

5. Verfahren nach Ansprüchen 1 bis 4, **dadurch gekennzeichnet, dass** man 2 bis 10 Äquivalente Chlorierungsmittel pro Mol methyl-substituiertes Biphenylcarbonitril der Formel (IV) einsetzt.

6. Verfahren nach Ansprüchen 1 bis 5, **dadurch gekennzeichnet, dass** man die Umwandlung eines trichlormethyl-substituierten Biphenylcarbonitrils der Formel (III) in die entsprechende Trifluormethyl-Verbindung durch Umsetzung mit wasserfreier Flusssäure durchführt.

7. Verfahren nach Anspruch 6, **dadurch gekennzeichnet, dass** man pro Mol trichlormethyl-substituiertes Biphenylcarbonitril 0 bis 0,2 Mol eines Fluorierungskatalysators ausgewählt aus der Gruppe Antimonpentafluorid, Antimonpentachlorid, Bortrifluorid und Titantetrachlorid einsetzt.

8. Verfahren nach Ansprüchen 1 bis 7, **dadurch gekennzeichnet, dass** man eine alkalische Hydrolyse durchführt.

9. Verfahren nach Anspruch 8, **dadurch gekennzeichnet, dass** man aus dem nach der alkalischen Hydrolyse vorliegenden Reaktionsgemisch zunächst gegebenenfalls vorhandene feste Bestandteile abtrennt, dass dann vorliegende wässrige Filtrat mit einem nicht mit Wasser mischbaren organischen Lösungsmittel extrahiert und aus der extrahierten wässrigen Phase durch Zusatz einer Säure die trifluormethyl-substituierten Biphenylcarbonsäuren der Formel (I) erhält.

10. Verbindungen der Formeln (II) und (III) 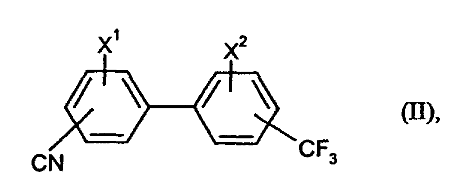 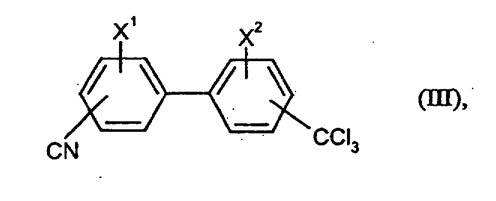 in denen
X¹ und X² gleich oder verschieden sind und jeweils für Chlor oder Fluor stehen.

11. 4'-Trichlormethyl-2-cyano-biphenyl.

## Claims

1. Process for the preparation of trifluoromethyl-substituted biphenylcarboxylic acids of the formula (I) 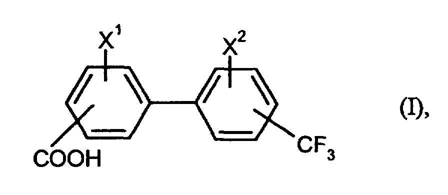 in which
X¹ and X² are identical or different and in each case represent hydrogen, chlorine or fluorine,
which is **characterized in that** methyl-substituted biphenylcarbonitriles of the formula (IV) 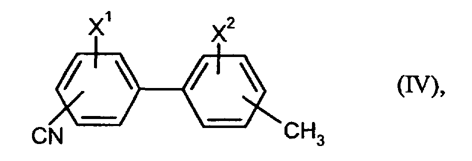 in which
X¹ and X² have the meanings stated for formula (I),
are converted into the corresponding trichloromethyl-substituted biphenylcarbonitriles of the formula (III) 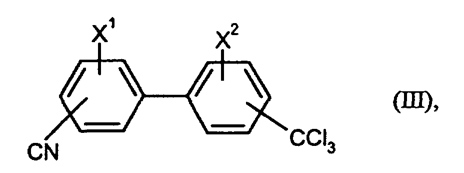 in which
X¹ and X² have the meanings stated for formula (I),
the trichloromethyl group thereof is converted into a trifluoromethyl group, and thus trifluoromethyl-substituted biphenylcarbonitriles of the formula (II) are obtained 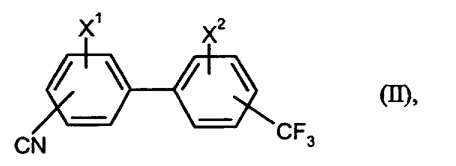 in which
X¹ and X² have the meanings stated for formula (I),
and finally the corresponding carboxylic acids are obtained therefrom by hydrolysis.

2. Process according to Claim 1, **characterized in that** X¹ and X² in the formulae (I) to (IV) represent hydrogen.

3. Process according to Claims 1 and 2, **characterized in that** the trifluoromethyl group in the formulae (I) and (II), the trichloromethyl group in formula (III) and the methyl group in formula (IV) are located in the position para to the biphenyl linkage and the carboxyl group in formula (I) and the nitrile group in the formulae (II), (III) and (IV) are located in the position ortho to the biphenyl linkage.

4. Process according to Claims 1 to 3, **characterized in that** the preparation of the trichloromethyl-substituted biphenylcarbonitriles of the formula (III) is carried out in the form of a free-radical side-chain chlorination at temperatures of from 80 to 250°C, while irradiating with a light source and/or with addition of a radical initiator and using chlorine as chlorinating agent.

5. Process according to Claims 1 to 4, **characterized in that** 2 to 10 equivalents of chlorinating agent are employed per mole of methyl-substituted biphenylcarbonitrile of the formula (IV).

6. Process according to Claims 1 to 5, **characterized in that** the conversion of a trichloromethyl-substituted biphenylcarbonitrile of the formula (III) into the corresponding trifluoromethyl compound is carried out by reaction with anhydrous hydrofluoric acid.

7. Process according to Claim 6, **characterized in that** 0 to 0.2 mol of a fluorination catalyst selected from the group of antimony pentafluoride, antimony pentachloride, boron trifluoride and titanium tetrachloride is employed per mole of trichloromethyl-substituted biphenylcarbonitrile.

8. Process according to Claims 1 to 7, **characterized in that** an alkaline hydrolysis is carried out.

9. Process according to Claim 8, **characterized in that** firstly solid constituents which are present where appropriate are removed from the reaction mixture after the alkaline hydrolysis, **in that** the resulting aqueous filtrate is extracted with a water-immiscible organic solvent, and the trifluoromethyl-substituted biphenylcarboxylic acids of the formula (I) are obtained from the extracted aqueous phase by addition of an acid.

10. Compounds of the formulae (II) and (III) 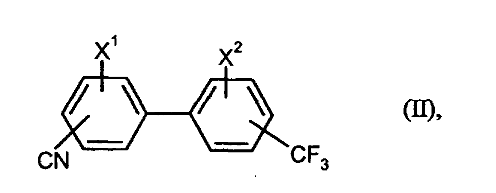 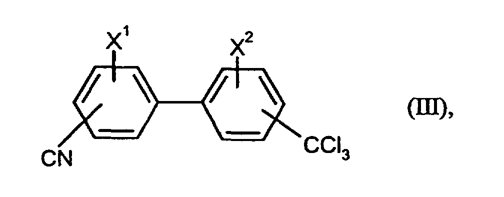 in which
X¹ and X² are identical or different and each represent chlorine or fluorine.

11. 4'-Trichloromethyl-2-cyanobiphenyl.

## Revendications

1. Procédé pour la préparation d'acides biphényle-carboxyliques à substituants trifluorométhyle de formule (I) 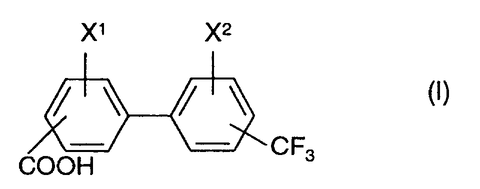 dans laquelle
X¹ et X², ayant des significations identiques ou différentes, représentent chacun l'hydrogène, le chlore ou le fluor,
**caractérisé en ce que** l'on convertit des biphényle-carbonitriles à substituants méthyle de formule (IV) 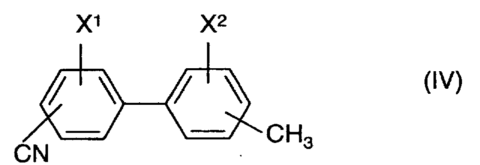 dans laquelle
X¹ et X² ont les significations indiquées en référence à la formule (I),
en les biphényle-carbonitriles correspondants à substituants trichlorométhyle de formule (III) 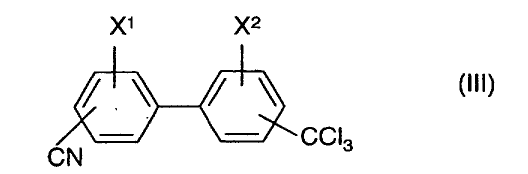 dans laquelle
X¹ et X² ont les significations indiquées en référence à la formule (I),
on convertit le groupe trichlorométhyle de ces derniers en un groupe trifluorométhyle, ce qui donne les biphényle-carbonitriles à substituants trifluorométhyle de formule (II) 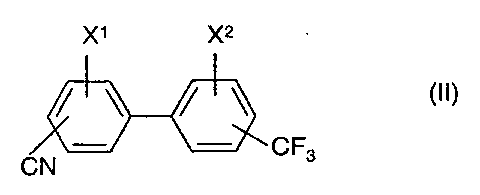 dans laquelle
X¹ et X² ont les significations indiquées en référence à la formule (I),
d'où une hydrolyse donne les acides carboxyliques correspondants.

2. Procédé selon la revendication 1, **caractérisé en ce que**, dans les formules (I) à (IV), X¹ et X² représentent l'hydrogène.

3. Procédé selon les revendications 1 et 2, **caractérisé en ce que** le groupe trifluorométhyle des formules (I) et (II), le groupe trichlorométhyle de la formule (III) et le groupe méthyle de la formule (IV) sont en position para de la liaison entre les groupes phényle et le groupe carboxyle de la formule (I) et le groupe nitrile des formules (II), (III) et (IV) se trouve en position ortho de la liaison entre les groupes phényle.

4. Procédé selon les revendications 1 à 3, **caractérisé en ce que** la préparation des biphényle-carbonitriles à substituants trichlorométhyle de formule (III) est réalisée sous la forme d'une chloruration radicalaire en chaîne latérale à des températures de 80 à 250°C, sous irradiation par une source de lumière et/ou avec adjonction d'un inducteur radicalaire et avec le chlore en tant qu'agent chlorant.

5. Procédé selon les revendications 1 à 4, **caractérisé en ce que** l'on utilise de 2 à 10 équivalents de l'agent chlorant par mole du biphényle-carbonitrile à substituant méthyle de formule (IV).

6. Procédé selon les revendications 1 à 5, **caractérisé en ce que** le biphényle-carbonitrile à substituant trichlorométhyle de formule (III) est converti en le dérivé correspondant trifluorométhylé par réaction avec l'acide fluorhydrique anhydre.

7. Procédé selon la revendication 6, **caractérisé en ce que**, pour 1 mol du biphényle-carbonitrile à substituant trichlorométhyle, on utilise en quantité de 0 à 0,2 mol un catalyseur de fluoration choisi parmi le pentafluorure d'antimoine, le pentachlorure d'antimoine, le trifluorure de bore et le tétrachlorure de titane.

8. Procédé selon les revendications 1 à 7, **caractérisé en ce que** l'on procède à une hydrolyse alcaline.

9. Procédé selon la revendication 8, **caractérisé en ce que**, à partir du mélange de réaction obtenu après hydrolyse alcaline, on sépare d'abord les constituants solides éventuels, on extrait ensuite le filtrat aqueux par un solvant organique non miscible à l'eau et à partir de la phase aqueuse extraite, par addition d'un acide, on obtient l'acide biphényle-carboxylique à substituant trifluorométhyle de formule (I).

10. Composés de formules (II) et (III) 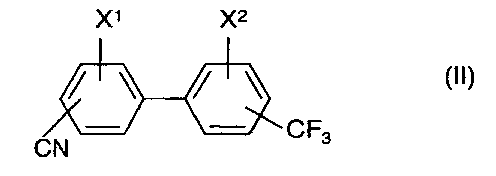 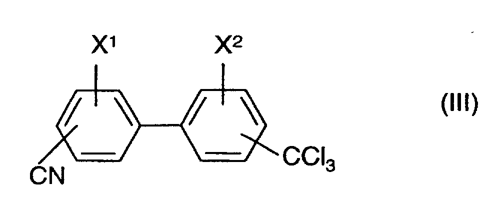 dans lesquelles
X¹ et X², ayant des significations identiques ou différentes, représentent chacun le chlore ou le fluor.

11. Le 4'-trichlorométhyl-2-cyano-biphényle.
